# FASCICULE DE BREVET EUROPEEN

(11) **EP 2 343 275 B1**
(45) Date de publication et mention de la délivrance du brevet: **24.04.2013**
(21) Numéro de dépôt: 11163602.3
(22) Date de dépôt: 18.12.2003
(51) Int. Cl.: C07C 315/04, C07C 315/06

(54) **Forme cristalline des énantiomères optiques du modafinil.**
Kristalline Form von den optischen Enantiomeren von Modafinil
Crystalline form of the optical enantiomers of modafinil

(30) Priorité: 20.12.2002 FR 0216412
(43) Date de publication de la demande: 13.07.2011
(62) Demande divisionnaire de: 03799631.1
(73) Titulaire: Teva Santé, 92931 La Défense Cedex (FR)
(72) Inventeur: Neckebrock, Olivier, 77340, PONTAULT COMBAULT (FR); Courvoisier, Laurent, 60290, LAIGNEVILLE (FR); Graf, Stéphanie, 95270, BELLOY EN FRANCE (FR); Serrure, Gilles, 78540 VERNOUILLET (FR); Coquerel, Gérard, 76520, BOOS (FR); Rose, Sébastien, 60190, CERNOY (FR); Besselievre, Christine, 91440, BURES SUR YVETTE (FR); Mallet, Franck, SG29AT HERTFORDSHIRE (GB); Van Langevelde, Adriaan Jan, 1325 NG, ALMERE (NL); Leproust, Pierre, 94000, CRETEIL (FR)
(74) Mandataire: Hallybone, Huw George

(56) Documents cités:
- EP-B- 0 720 595
- WO-A-02/10125
- US-A- 4 177 290
- US-A- 4 927 855

## Description

L'invention concerne des formes cristallines des énantiomères du modafinil.

En particulier, la présente invention est relative à une forme polymorphique des énantiomères dextrogyre et lévogyre du modafinil, désignée forme I, telle que caractérisée dans les revendications.

Est également décrit un nouveau procédé de préparation des énantiomères optiques du modafinil à partir de l'acide (±) modafinique.

Le brevet US 4,177,290 décrit le modafinil sous forme racémique, encore dénommé (±) 2-(benzhydrylsulfinyl)acétamide ou (±) 2-[(di-phénylméthyl)sulfinyl] acétamide, à titre de composé ayant des propriétés stimulantes du système nerveux central.

Le brevet US 4,927,855 décrit les deux énantiomères optiques du modafinil. Il décrit plus particulièrement l'énantiomère lévogyre et son utilisation en tant qu'agent antidépresseur ou stimulant dans le traitement de l'hypersomnie et des troubles liés à la maladie d'Alzheimer. Le procédé de préparation des deux énantiomères optiques du modafinil à partir de l'acide (±) modafinique ou acide (±)-benzhydrylsulfinylacétique décrit dans ce document est représenté dans le schéma de synthèse suivant :

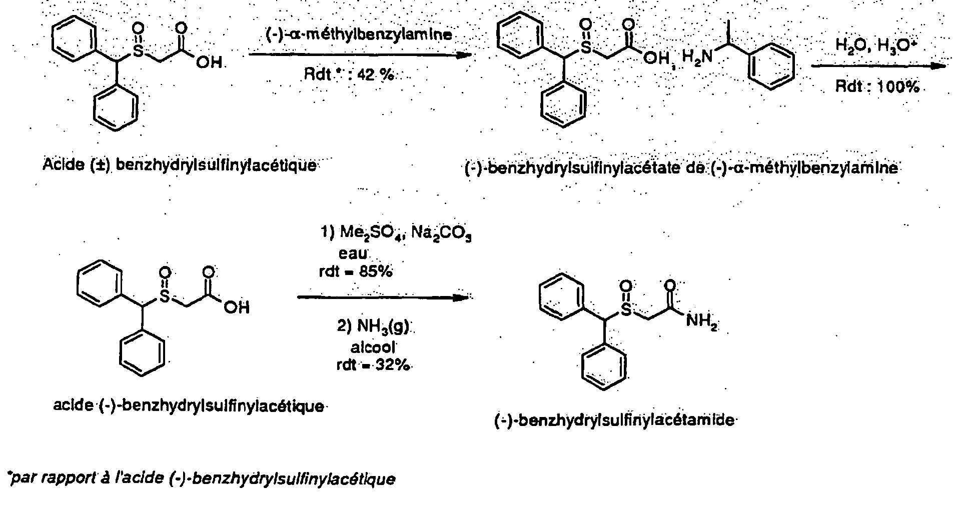

Ce procédé consiste à réaliser, dans une première étape, un dédoublement des énantiomères optiques de l'acide (±) modafinique via la formation de diastéréoisomères avec l'agent optiquement actif α-méthylbenzylamine.

Le (-)-benzhydrylsulfinylacétate de (-)-α-méthylbenzylamine est ensuite converti par hydrolyse acide en acide (-)-benzhydrylsulfinylacétique. Celui-ci est estérifié en présence de diméthyl sulfate puis amidifié en présence d'ammoniac (gaz). L'énantiomère (-) ou I (lévogyre) du modafinil est obtenu par ce procédé avec un rendement global de 5,7% par rapport à l'acide (±) modafinique, calculé sur la base des rendements correspondant à chaque étape.

Le terme "énantiomère" désigne des molécules stéréoisomères qui sont des images en miroir l'une de l'autre non superposables. Les énantiomères sont typiquement désignés soit par (+) et (-) soit par (d) et (I), ce qui indique un pouvoir rotatoire optique au centre chiral.

La stéréoisomérie peut également être notée soit par (D) ou (L) soit par (R) et (S), qui sont des descriptifs de la configuration absolue.

Dans ce qui suit, l'énantiomère lévogyre du modafinil sera indifféremment désigné énantiomère I ou (-), l'énantiomère dextrogyre étant pour sa part désigné énantiomère d ou (+).

Il a maintenant été découvert un procédé permettant d'obtenir différentes formes cristallines des énantiomères optiques du modafinil. De façon plus spécifique, les inventeurs ont montré que la forme cristalline obtenue dépendait principalement de la nature du solvant de recristallisation mis en oeuvre.

Le terme "forme cristalline" désigne indifféremment, au sens de la présente description, une forme polymorphique ou un solvate.

Par "forme polymorphique", on entend une structure organisée n'impliquant que des molécules de soluté, possédant une empreinte cristalline caractéristique.

Le terme "solvate" désigne une structure organisée possédant une empreinte cristalline caractéristique impliquant à la fois des molécules de soluté et des molécules de solvant. Les solvates impliquant une molécule de soluté pour une molécule de solvant sont appelés solvates vrais.

Une forme polymorphique ici décrite est désignée forme I, laquelle correspond à la forme polymorphique thermodynamiquement la plus stable, dans les conditions normales de température et de pression.

La forme I présente le spectre de diffraction X ci-dessous dans lequel d représente la distance réticulaire et le rapport (I/Io) l'intensité relative.

| CRL 40982 FORME I | | |
|---|---|---|
| 2 Theta (degrés) | d (Å) | I/lo (%) |
| 9.8 | 13.40 | 32 |
| 15.4 | 8.54 | 87 |
| 20.8 | 6.34 | 24 |
| 26.4 | 5.01 | 14 |
| 28.3 | 4.68 | 19 |
| 28.7 | 4.62 | 16 |
| 29.9 | 4.44 | 45 |
| 31.1 | 4.27 | 100 |
| 31.6 | 4.20 | 23 |
| 32 | 4.15 | 14 |
| 33.1 | 4.02 | 78 |
| 33.4 | 3.98 | 84 |
| 34.1 | 3.90 | 16 |
| 35.1 | 3.80 | 15 |
| 39 | 3.43 | 22 |

| | | |
|---|---|---|
| Diffractomètre: Miniflex Rigaku (Elexience) | | |

Les formes cristallines d'un composé donné présentent généralement des propriétés physiques, pharmaceutiques, physiologiques et biologiques très distinctes les unes des autres.

En ce sens, les formes cristallines du modafinil optiquement actif, en particulier les formes polymorphiques, sont intéressantes en ce qu'elles présentent des caractéristiques avantageuses et différentes par rapport à la forme I.

Il a maintenant été découvert, selon un autre aspect de la divulgation, un nouveau procédé de préparation des énantiomères optiques du modafinil à partir de l'acide (±)-modafinique, ledit procédé permettant d'isoler chaque énantiomère avec des rendements et une pureté optique nettement supérieurs à ceux décrits dans le brevet US 4,927,855.

De façon particulièrement avantageuse, il a maintenant été mis au point un procédé de dédoublement des deux énantiomères optiques de l'acide (±)-modafinique par cristallisation préférentielle, avantageusement applicable à l'échelle préparative.

Ce procédé de dédoublement de l'acide (±)-modafinique présente de nombreux avantages :
- il évite l'utilisation d'un agent chiral intermédiaire onéreux dont la préparation ultérieure implique des pertes rarement inférieures à 10% (De Min., M., Levy, G. et Micheau J.-C., 1988 ; J. Chem. Phys. 85, 603-19) ;
- les deux énantiomères sont obtenus directement, contrairement à la méthode mettant en oeuvre le dédoublement classique par formation de sels diastéréoisomères ;
- le rendement est théoriquement quantitatif par suite des recyclages successifs des eaux-mères ;
- la purification des cristaux d'énantiomères bruts est aisée.
La divulgation vise donc à fournir un procédé de préparation des formes cristallines des énantiomères du modafinil.

La divulgation a également pour but de proposer un nouveau procédé de préparation des énantiomères optiques du modafinil, et notamment de l'énantiomère lévogyre du modafinil.

### • PROCEDE DE PREPARATION DE LA FORME POLYMORPHIQUE I DU L-MODAFINIL

Ces buts et d'autres sont atteints par la présente divulgation qui vise plus précisément, selon un premier aspect, un procédé de préparation de la forme cristalline I des énantiomères optiques du modafinil, comprenant les étapes suivantes :
i) dissoudre l'un des énantiomères optiques du modafinil dans un solvant autre que l'éthanol ;
ii) cristalliser ledit énantiomère du modafinil ; et
iii) récupérer la forme cristalline dite forme I de l'énantiomère du modafinil ainsi obtenue.
Au sens de la présente divulgation, le solvant mis en oeuvre à l'étape i) du procédé, encore dénommé "solvant de recristallisation" est un solvant capable d'assurer la cristallisation dudit énantiomère optique du modafinil, de préférence à la pression atmosphérique. Il s'agit, en d'autres termes, de tout solvant A capable de former, à pression donnée, avec au moins l'un des énantiomères
- dans un premier domaine de température et de concentration, un système monophasé comprenant au moins l'un des énantiomères en solution diluée dans le solvant A ;
- dans un deuxième domaine de température et de concentration distinct du précédent, un deuxième système biphasé comprenant des cristaux dudit énantiomère en présence de solution saturée, les deux domaines étant séparés l'un de l'autre par la courbe de solubilité dudit énantiomère T (°C) = f (concentration en énantiomère) à la pression considérée.

En général, la cristallisation de l'étape ii) consiste à passer du système monophasé au système biphasé en faisant varier la température et la concentration.

A titre illustratif et non limitatif de solvants pouvant convenir au procédé de recristallisation selon La divulgation, on peut notamment citer les solvants alcooliques, les solvants esters d'acide carboxylique, les solvants éthérés, les solvants chlorés, les solvants aromatiques, les solvants cétoniques aliphatiques inférieurs. D'autres solvants sont, par exemple, les solvants acides carboxyliques, les solvants polaires non protiques, les hydrocarbures alicycliques, les hydrocarbures aliphatiques, les carbonates, les hétéroaromatiques, l'eau.

Parmi les solvants alcooliques, on peut citer notamment les alcools d'alkyles inférieurs tels que le méthanol, l'éthanol, le propanol, l'isopropanol, le butanol, l'isobutanol, le 2-méthyl-2-pentanol, le 1,2-propanediol, l'alcool de t-amyle, le méthanol, le propanol et l'isopropanol étant particulièrement préférés.

Parmi les solvants de type esters d'acide carboxylique, on peut citer notamment les acétates d'alkyle tels que l'acétate de méthyle, l'acétate d'éthyle, l'acétate de n-propyle, l'acétate d'isopropyle, l'acétate de n-butyle, les formates d'alkyle tels que le formate d'éthyle, l'acétate d'éthyle étant particulièrement préféré.

Sont utiles à titre de solvants éthérés de recristallisation, le diéthyléther, le tétrahydrofuranne (THF), le dioxanne, le dibutyléther, l'isopropyléther, le t-butylméthyléther, le tétrahydropyrane, le tétrahydrofuranne étant particulièrement préféré.

Parmi les solvants chlorés, on peut citer les hydrocarbures chlorés, notamment le chloroforme, le 1,2-dichloroéthane, le dichlorométhane et les aromatiques chlorés tels que le chlorobenzène.

Comme exemples de solvants aromatiques, on peut citer l'ortho, le méta, le paraxylène ou un mélange d'ortho, de méta et paraxylène, le méthoxybenzène, le nitrobenzène, le trifluorotoluène, le toluène, l'ortho, le méta et le paraxylène étant particulièrement préférés.

Sont utiles à titre de solvants cétoniques les solvants tels que l'acétone, la méthyléthylcétone, la méthylisobutylcétone, la butan-2-one, la cyclopentanone, l'isobutylméthylcétone, la 2-pentanone, la 3-pentanone.

Comme exemple de solvant acide carboxylique, on peut mentionner en particulier l'acide acétique.

A titre d'exemple de solvant hétéroaromatique, on peut citer en particulier la pyridine.

Des exemples de solvants polaires non protiques sont notamment l'acétonitrile, le propionitrile, la 4-méthylmorpholine, la N,N-dimé-thylacétamide, le nitrométhane, la triéthylamine, la N-méthyl-pyrrolidone (NMP).

Des exemples d'hydrocarbures aliphatiques sont notamment l'heptane, le 2,2-4-triméthylpentane.

Des exemples d'hydrocarbures alicycliques sont notamment le cyclopentane, le cyclohexane.

Des exemples de carbonates sont notamment les alkylcarbonates tels que le diméthylcarbonate.

Selon un mode de réalisation préférentiel du procédé selon la divulgation, les solvants de cristallisation sont choisis parmi l'acétone, le méthanol, le dioxanne 1-4, l'acétate d'éthyle, les mélanges d'ortho, méta, paraxylène, l'isopropanol, le n-propanol, le diméthylcarbonate, le tétrahydrofuranne, le chloroforme et la méthyléthylcétone, l'eau et les mélanges alcool/H₂O.

Ainsi, les formes cristallines des énantiomères optiques du modafinil peuvent être obtenues par recristallisation des énantiomères dans certains solvants, dont la nature et éventuellement les conditions de cristallisation déterminent principalement le type de forme cristalline obtenue.

Le solvant de recristallisation, par son interaction avec les groupements fonctionnels et les substituants électro-attracteurs ou électro-donneurs, peut en effet favoriser certains arrangements moléculaires qui donneront naissance à une forme cristalline déterminée dans des conditions de cristallisation données. Généralement, on chauffe le solvant de recristallisation mis en oeuvre à l'étape i), notamment au reflux, jusqu'à obtenir la dissolution complète de l'énantiomère optique du modafinil dans le solvant. Si la concentration de l'énantiomère optique du modafinil à l'étape i) ne constitue pas un facteur critique de la cristallisation, on préfère cependant opérer en présence d'une concentration en énantiomère optique du modafinil proche de la concentration de saturation dans le solvant de recristallisation considéré.

Selon un mode de réalisation de la divulgation, l'énantiomère optique du modafinil est dissout en chauffant le solvant à reflux et une quantité supplémentaire dudit énantiomère optique est ensuite ajoutée par fractions de manière à atteindre la saturation. Du solvant supplémentaire peut être rajouté pour assurer une dissolution complète. Selon un autre mode de réalisation de la divulgation, l'énantiomère optique du modafinil est mis en suspension dans le solvant chauffé au reflux, et une quantité supplémentaire de solvant est ensuite ajoutée par fractions de manière à obtenir une solution homogène et atteindre ainsi la saturation.

Le processus de cristallisation de l'énantiomère optique du modafinil à l'étape ii) peut être accéléré selon des techniques connues de l'homme du métier, à savoir le refroidissement de la solution, l'évaporation d'une partie du solvant, l'addition d'un anti-solvant ou l'ensemencement de la solution avec des cristaux de modafinil optiquement actif de même forme cristalline que celle attendue. Le plus souvent, le mélange est maintenu sous agitation tout au long du processus de cristallisation, de manière à obtenir une suspension homogène et un renouvellement rapide de la liqueur-mère autour de chaque cristallite.

Le processus de cristallisation du procédé selon la divulgation peut être réalisé dans des conditions thermodynamiques ou cinétiques.

Au sens de la présente description, on entend par "cristallisation dans des conditions thermodynamiques" une cristallisation réalisée dans des conditions où l'équilibre entre la solution homogène, d'une part, et la solution saturée en présence de cristaux de I- ou d-modafinil, d'autre part, est maintenu.

A titre d'exemple, une cristallisation thermodynamique peut être réalisée en refroidissant lentement la solution obtenue à l'étape i), typiquement en laissant refroidir la solution à température ambiante ou en appliquant une vitesse ou une rampe de refroidissement inférieure ou égale à 0,75°C/mn, de préférence à 0,6°C et plus préférentiellement à 0,5°C/mn.

On entend par "cristallisation réalisée dans des conditions cinétiques", au sens de la présente description, une cristallisation dans laquelle l'équilibre entre la solution homogène, d'une part, et la solution saturée en présence de cristaux de d ou I-modafinil, d'autre part, est déplacé brutalement vers ce dernier domaine biphasé, i. e. vers la formation de cristaux.

A titre illustratif, une cristallisation dite cinétique peut être réalisée notamment par refroidissement rapide, par exemple en appliquant une rampe de refroidissement de 300°C/mn, ou bien par précipitation par ajout d'un antisolvant de la solution obtenue à l'étape i).

A titre illustratif et non limitatif, ces deux types de cristallisation thermodynamique ou cinétique sont réalisés dans la présente description par refroidissement lent ou rapide.

Bien entendu, toute autre technique de cristallisation telle que l'évaporation du solvant ou la précipitation, permettant de se placer dans des conditions cinétiques et/ou thermodynamiques, entre également dans le cadre du procédé selon la divulgation.

Ainsi, selon un mode de réalisation particulier de la divulgation, la cristallisation à l'étape ii) peut être réalisée par précipitation, éventuellement en présence de germes de cristaux de la forme cristalline désirée.

Les inventeurs ont en outre montré que certains solvants peuvent conduire à des formes cristallines, plus spécifiquement à des formes polymorphiques, distinctes selon que la cristallisation est réalisée dans des conditions cinétiques ou thermodynamiques.

Selon un mode de réalisation privilégié de la divulgation, la cristallisation consiste en un refroidissement de la solution obtenue à l'étape i).

Le cas échéant, selon un premier mode, le refroidissement est rapide et correspond généralement à un trempage de la solution obtenue à l'étape i) dans un bain de température inférieure ou égale à 0°C tel qu'un bain d'eau glacé pendant un temps suffisant pour permettre une cristallisation complète de la solution, ou bien encore à un refroidissement avec une rampe de refroidissement comprise par exemple entre -1°C et -5°C/mn.

Selon un deuxième mode de réalisation de la divulgation, le refroidissement est lent. Dans ce cadre, on laisse généralement la solution refroidir depuis la température de reflux du solvant jusqu'à température ambiante ou bien on refroidit la solution avec une rampe de refroidissement comprise de préférence entre - 0,1 °C/mn et - 0,8°C/mn, et plus préférentiellement proche de - 0,5°C/mn, jusqu'à généralement une température de 15° à 20 °C.

Parmi les combinaisons de solvants / antisolvants préférées selon la divulgation, on peut citer notamment les combinaisons eau / acétone, acétonitrile / eau, éthanol / eau, méthanol / eau, acide acétique / eau.

Enfin, les formes cristallines des énantiomères optiques du modafinil peuvent être isolées selon des méthodes classiques telles que la filtration et la centrifugation.

A titre illustratif et non limitatif, le procédé de préparation selon la divulgation est mis plus particulièrement en oeuvre avec l'énantiomère lévogyre du modafinil.

Selon un mode de réalisation particulier de la divulgation, la forme cristalline obtenue selon ce procédé est une forme polymorphique.

On notera à ce sujet que, de façon générale, chacun des énantiomères (I) et (d) d'un composé chimique donné conduisent, lorsqu'ils sont recristallisés dans les mêmes conditions expérimentales, à des formes cristallines, notamment polymorphiques, ayant des spectres de diffraction X réalisés sur poudre identiques.

A ce propos, on se réfèrera notamment à l'ouvrage de J. Bernstein « Polymorphism in molecular crystals » 2002, University Press, Oxford, UK, et à la publication de G. Coquerel, Enantiomer, 2000; 5(5): 481-498 ; Gordon and Breach Science Publishers.

Pour ce motif, la forme dextrogyre dont les spectres de diffraction X des formes cristallines sont identiques à ceux de la forme lévogyre exposés ci-après et réciproquement font partie de l'invention.

Dans ce qui suit, la forme polymorphique désignée forme I couvre ainsi la forme CRL40982 forme I obtenue à partir de l'énantiomère lévogyre et la forme CRL40983 forme I obtenue à partir de l'énantiomère dextrogyre.

### Forme I

Dans ce cadre, le procédé mettant en oeuvre un solvant choisi parmi l'acétone, l'éthanol, le dioxanne 1-4, l'acétate d'éthyle et les mélanges d'ortho, méta, paraxylène, et une étape de cristallisation par refroidissement lent conduit à l'obtention de la forme I ou CRL40982 forme I.

Le procédé mettant en oeuvre un solvant choisi parmi le méthanol, l'eau ou les mélanges alcool/eau, en particulier méthanol/eau et éthanol/eau, ainsi qu'une étape de cristallisation par refroidissement rapide conduit à l'obtention de la forme I ou CRL 40982 Forme I.

Selon une autre variante également préférée de la divulgation, le procédé mettant en oeuvre le méthanol et une étape de cristallisation par précipitation par ajout d'eau froide comme antisolvant du méthanol conduit à la forme I.

### • FORMES POLYMORPHIQUES DU (-)-MODAFINIL

La divulgation a également pour objet le solvate de diméthylcarbonate du (-)-modafinil, caractérisé par le spectre de diffraction suivant dans lequel d représente la distance réticulaire et I/lo l'intensité relative :

| SOLVATE DE DIMETHYLCARBONATE | | |
|---|---|---|
| 2 Theta (degrés) | d (Å) | I/lo (%) |
| 7,17 | 12,31 | 38 |
| 9,12 | 9,69 | 29 |
| 9,72 | 9,09 | 16 |
| 10,35 | 8,54 | 35 |
| 12,17 | 7,27 | 100 |
| 14,25 | 6,21 | 16 |
| 16,26 | 5,45 | 10 |
| 17,36 | 5,10 | 13 |
| 17,72 | 5,00 | 21 |
| 18,35 | 4,83 | 9 |
| 19,16 | 4,63 | 9 |
| 19,88 | 4,46 | 14 |
| 21,04 | 4,22 | 12 |
| 21,49 | 4,13 | 25 |
| 21,73 | 4,09 | 24 |
| 23,49 | 3,78 | 22 |
| 24,55 | 3,62 | 35 |
| 25,24 | 3,53 | 8 |
| 26,05 | 3,42 | 9 |
| 26,88 | 3,32 | 7 |
| 27,48 | 3,24 | 13 |
| 27,81 | 3,21 | 10 |
| 28,79 | 3,10 | 8 |

| | | |
|---|---|---|
| Diffractomètre : Siemens AG. | | |

Selon un autre aspect, la divulgation a également pour objet un procédé de conversion d'une première forme cristalline d'un des énantiomères du modafinil en une deuxième forme cristalline distincte de la première, ledit procédé comprenant les étapes consistant à :
i) suspendre la forme cristalline dudit énantiomère du modafinil dans un solvant ;
ii) récupérer la forme cristalline obtenue.

A titre de solvants pouvant convenir à ce procédé, on peut notamment citer l'acétonitrile.

De façon générale, la forme cristalline initiale est maintenue en suspension à une température inférieure à la température d'homogénéisation, pendant une durée suffisante pour permettre la conversion totale de la forme initiale. Cette durée peut varier notamment selon la nature du solvant, de la forme cristalline initiale, de la température du milieu. De façon classique, la forme cristalline est maintenue en suspension pendant au moins 24 heures à température ambiante, sous pression atmosphérique, le plus souvent pendant 72 heures environ.

A titre illustratif, ce procédé est mis en oeuvre avec le (-)-modafinil.

Dans ce cadre, selon un mode de réalisation particulier de la divulgation, le procédé met en oeuvre la forme I dans l'acétonitrile à l'étape i), ce par quoi on obtient un solvate d'acétonitrile du (-)-modafinil.

A titre indicatif, la forme I est maintenue en suspension pendant plusieurs jours, de préférence pendant 3 jours à température ambiante, à pression atmosphérique.

La divulgation a également pour objet le solvate d'acétonitrile du (-)-modafinil susceptible d'être obtenu selon le procédé de recristallisation de la divulgation. Il est caractérisé par le spectre de diffraction suivant dans lequel d représente la distance réticulaire et I/lo l'intensité relative :

| SOLVATE D'ACETONITRILE | | |
|---|---|---|
| 2 Theta (degrés) | d (Å) | I/lo (%) |
| 5,46 | 16,17 | 46 |
| 6,25 | 14,14 | 95 |
| 7,17 | 12,32 | 51 |
| 8,28 | 10,66 | 81 |
| 9,02 | 9,79 | 68 |
| 9,51 | 9,29 | 53 |
| 10,34 | 8,54 | 53 |
| 10,84 | 8,15 | 63 |
| 11,33 | 7,80 | 79 |
| 12,47 | 7,09 | 53 |
| 14,02 | 6,31 | 45 |
| 15,20 | 5,83 | 35 |
| 15,76 | 5,62 | 34 |
| 16,37 | 5,41 | 40 |
| 17,37 | 5,10 | 51 |
| 18,10 | 4,90 | 46 |
| 19,05 | 4,66 | 44 |
| 19,36 | 4,58 | 37 |
| 19,89 | 4,46 | 39 |
| 20,48 | 4,33 | 59 |
| 21,14 | 4,20 | 55 |
| 22,10 | 4,02 | 100 |
| 22,65 | 3,92 | 60 |
| 23,17 | 3,835 | 42 |
| 23,89 | 3,72 | 33 |
| 24,72 | 3,60 | 38 |
| 24,93 | 3,57 | 37 |
| 25,81 | 3,45 | 37 |
| 26,73 | 3,33 | 55 |
| 27,52 | 3,24 | 30 |
| 27,97 | 3,19 | 30 |
| 28,89 | 3,09 | 31 |
| 29,44 | 3,03 | 27 |

| | | |
|---|---|---|
| Diffractomètre : Siemens AG. | | |

### • COMPOSITIONS PHARMACEUTIQUES COMPRENANT LA FORME POLYMORPHIQUE I DU (-)-MODAFINIL, DU (+)-MODAFINIL RESPECTIVEMENT

L'invention vise également les compositions pharmaceutiques comprenant la forme polymorphique CRL 40982 forme I du (-)-modafinil, CRL 40983 forme I respectivement, éventuellement en association avec un véhicule pharmaceutiquement acceptable.

Ces compositions peuvent être administrées par voie orale, par voie muqueuse (par exemple, oculaire, intranasale, pulmonaire, gastrique, intestinale, rectale, vaginale, ou bien via l'appareil urinaire) ou par voie parentérale (par exemple, sous-cutanée, intradermique, intramusculaire, intraveineuse, ou intrapéritonéale). Selon un mode préférentiel, les compositions pharmaceutiques selon l'invention sont administrées par voie orale, sous forme de comprimés, de pilules, de gélules ou de granules à libération immédiate ou à libération contrôlée, sous forme de poudre, de capsules, de suspension dans un liquide ou dans un gel, d'émulsion, ou bien de lyophilisat, plus préférentiellement sous forme de comprimés, de capsules, de suspension dans un liquide ou dans un gel. Le véhicule d'administration peut comprendre un ou plusieurs excipients pharmaceutiquement acceptables qui sont susceptibles d'assurer la stabilité des formes polymorphiques (par exemple, une suspension d'un polymorphe dans une huile).

Les compositions pharmaceutiques selon l'invention comprennent la forme polymorphique du (-)-modafinil et du (+)-modafinil I, éventuellement en mélange avec les formes polymorphiques du (-)-modafinil et du (+)-modafinil II, III, IV ou V respectivement et/ou avec un ou plusieurs excipients pharmaceutiquement acceptables.

Une composition solide pour une administration par voie orale est préparée par addition au principe actif d'un ou de plusieurs excipients, notamment d'une charge, et, le cas échéant d'un liant, d'un agent délitant, d'un lubrifiant, d'un surfactant et d'un émulsifiant, d'un solubilisant, d'un colorant, d'un succédané de sucre ou d'un correcteur de goût et par mise en forme du mélange par exemple sous forme de comprimé ou de capsule.

Des exemples de charges englobent le lactose, le sucrose, le mannitol ou le sorbitol ; des préparations à base de cellulose, telles que par exemple de l'amidon de maïs, de l'amidon de riz, de l'amidon de pomme de terre.

Des exemples de liants englobent la gélatine, la gomme adragante, la méthylcellulose, l'hydroxypropylcellulose, la carboxyméthylcellulose de sodium, et/ou la polyvinyle pyrrolidone (PVP), la povidone, la copovidone, le dextrane, la dextrine, la cyclodextrine et ses dérivés tels que l'hydroxypropyl-β-cyclodextrine. Des exemples de succédanés de sucre englobent l'aspartame, la saccharine, le cyclamate de sodium.

Des exemples d'agents correcteurs de goût englobent le cacao en poudre, la menthe sous forme d'herbe, la poudre aromatique, la menthe sous forme d'huile, le bornéol et la cannelle en poudre.

Des exemples de surfactants et d'émulsifiants englobent en particulier le polysorbate 20, 60, 80, le sucroester (7-11-15), le poloxamère 188, 407, le PEF 300, 400 et le stéarate de sorbitane.

Des exemples d'agents solubilisants englobent le miglyole 810, 812, les glycérides et leurs dérivés, le propylène glycol.

Des exemples d'agents délitants englobent, par exemple, la polyvinyle pyrrolidone, la carmelose de sodium ou bien l'acide alginique ou un sel de celui-ci tel que l'alginate de sodium.

Des exemples d'agents lubrifiants englobent le stéarate de magnésium, le fumarate de magnésium stéarilé, l'acide béhénique, et ses dérivés.

Les compositions pharmaceutiques de la présente invention peuvent également contenir une autre forme cristalline du (-)-modafinil ou du (+)-modafinil, respectivement, dont notamment la forme I et un autre principe actif ou inactif en mélange avec un ou plusieurs autres formes polymorphiques du modafinil, telles que la forme III, la forme II, la forme IV et la forme V.

Au sens de la présente invention, l'expression "véhicule pharma-ceutiquement acceptable" couvre des solvants, des milieux de dispersion, des agents antifongiques et antibactériens, des agents isotoniques et des agents retardant l'absorption. L'utilisation de tels milieux et agents pour les substances pharmaceutiquement actives est bien connue de l'homme du métier.

Dans ce qui suit, les exemples sont donnés à titre illustratif de la présente invention.

### FIGURES (DE RÉFÉRENCE)

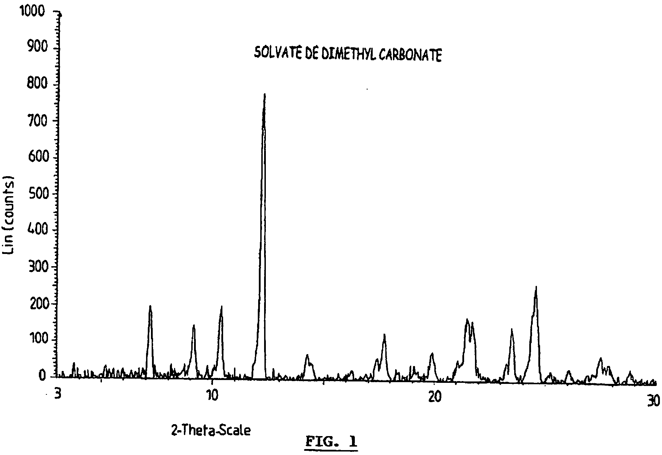

La Figure 1 représente le spectre de diffraction par rayons X réalisé sur poudre correspondant au solvate de diméthylcarbonate de l'énantiomère lévogyre, dextrogyre du modafinil respectivement (diffractomètre : Siemens AG).

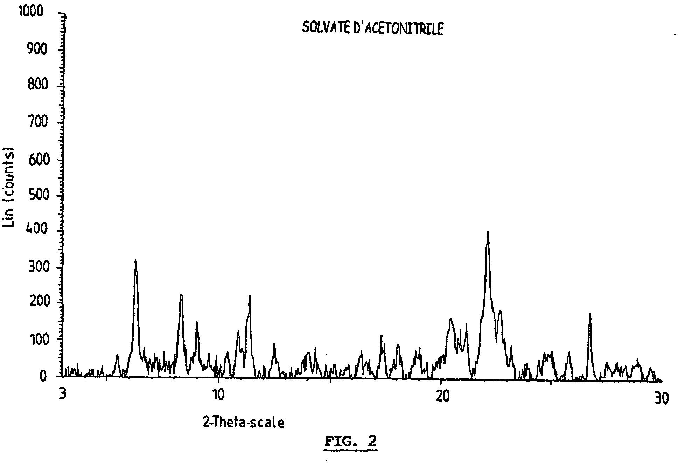

La Figure 2 représente le spectre de diffraction par rayons X réalisé sur poudre correspondant au solvate d'acétonitrile de l'énantiomère lévogyre, dextrogyre du modafinil respectivement (diffractomètre : Siemens AG).

### EXEMPLES

### PREPARATION DE LA FORME CRISTALLINE I DE L'ENANTIOMERE (-)-MODAFINIL, DU (+)-MODAFINIL RESPECTIVEMENT

### GÉNÉRALITÉS

Les nouvelles formes cristallines des énantiomères du modafinil ont été caractérisées respectivement par spectroscopie de diffraction par rayons X sur poudre, laquelle fournit une empreinte digitale unique, caractéristique de la forme cristalline étudiée et permet de différencier celle-ci des énantiomères du modafinil amorphes et de toute autre forme cristalline des énantiomères du modafinil.

Les données de diffraction aux rayons X ont été mesurées soit :
- en utilisant un système D5005 en tant que diffractomètre de poudre aux rayons X (Siemens AG, Karlsruhe, Allemagne, méthode d'analyse de données Eva 5.0), avec une radiation de cuivre filtrée au nickel de λ = 1.540 Å (avec une vitesse d'accélérateur de 40 KV, courant de tubes de 40 mA) avec une rotation de l'échantillon durant la mesure (angle : 3 à 40° [2 theta] ; à une vitesse de 0.04° [2 theta].s⁻¹, la taille du pas étant de 0.04° ; préparation de l'échantillon avec une orientation préférentielle).
- en utilisant un système Miniflex Rigaku (Elexience) en tant que diffractomètre de poudre aux rayons X, avec une radiation de chrome, une vitesse d'accélérateur de 30 KV, un courant de tubes de 15 mA et avec une rotation de l'échantillon durant la mesure (angle : 3 à 80° [2 theta] ; à une vitesse de 0.05° [2 theta]. s⁻¹, la taille du pas étant de 0.1° ; préparation de l'échantillon avec une orientation préférentielle).
- en utilisant un système GADDS en tant que diffractomètre de poudre aux rayons X (Bruker, the Netherlands), équipé avec un détecteur « Hi-Star area » et équipé pour l'analyse des plaques 96 puits. Les analyses sont effectuées à température ambiante en utilisant une radiation de cuivre CuKₐₗₚₕₐ dans la région des angles 2 théta compris entre 3 et 42°. Le spectre de diffraction pour chaque puit est collecté entre deux domaines de valeur d'angle 2 théta (3°≤ 2 Théta ≤ 21° et 19° ≤ 2 Théta ≤ 42°) avec un temps d'exposition entre 50 et 250 secondes.

Bien entendu, les valeurs d'intensité peuvent varier en fonction de la préparation de l'échantillon, du montage et des instruments de mesure. La mesure en 2 theta peut également être affectée par les variations liées aux instruments de mesure, si bien que les pics correspondants peuvent varier de ± 0,04°à de ± 0,2° selon l'appareillage. Aussi, l'homme du métier appréciera de pouvoir disposer des distances réticulaires qui constituent des données essentielles des spectres de diffraction. Les distances réticulaires sont calculées en utilisant la relation de Bragg [(2d sin theta = nλ, dans laquelle d = la distance réticulaire (Å), λ = la longueur d'onde de la radiation de cuivre, theta = l'angle de rotation du cristal (en degrés)] lorsque cette relation est satisfaite.

### EXEMPLES 1 A 10 : PREPARATION DE LA FORME I DU (-)-MODAFINIL, DU (+)-MODAFINIL RESPECTIVEMENT

### - Exemple 1 :

a) L'énantiomère I du modafinil a été solubilisé à reflux dans des solvants polaires : le méthanol, l'éthanol absolu, l'éthanol absolu contenant 3% d'eau, l'éthanol dénaturé au toluène (2.5%) et contenant 3% d'eau et l'eau, selon les conditions expérimentales détaillées dans le tableau 1.

**Tableau 1 :**

| Solvant | Quantité de I-modafinil (g) | Volume de solvant (ml) | Rendement % |
|---|---|---|---|
| Méthanol | 8,37 | ≤ 50 | 63 |
| Ethanol absolu | 7,85 | 115 | 56 |
| Ethanol absolu + 3% d'eau | 5 | 70 | 54 |
| Ethanol dénaturé au toluène+ 3% d'eau | 5 | 70 | 56 |
| Eau | 5 | ≥ 400 | 88 |

Après refroidissement rapide par trempe dans un bain d'eau et de glace pendant 30 minutes, le milieu a été filtré puis séché à l'étuve à 35°C. Le produit cristallisé a été identifié par son spectre de diffraction X sur poudre comme étant le polymorphe de forme I du I-énantiomère du modafinil.
b) L'énantiomère d du modafinil (555 g), traité dans les mêmes conditions expérimentales que l'exemple 1 a dans un mélange d'éthanol dénaturé au toluène (2 L) et d'eau (0,1 L), cristallise sous la forme polymorphique I telle qu'identifiée par son spectre de diffraction X sur poudre avec un rendement de 91 %.

### - Exemple 2 : Recristallisation dans l'acétone

a) 2 g de (-)-modafinil sont suspendues dans l'acétone (20 ml) dans un ballon tricol équipé d'un réfrigérant, d'un thermomètre et d'un agitateur. Le mélange est chauffé au reflux. Le mélange réactionnel est agité pendant 30 mn à environ 56°C jusqu'à dissolution complète du (-)-modafinil. La solution est alors refroidie lentement avec une vitesse de -0,5°C/mn jusqu'à 10°C sous agitation. Le mélange réactionnel est filtré, et le solide obtenu est séché pour conduire à la forme I du (-)-modafinil identifié par son spectre de Diffraction X. Rendement 62%.
b) Les mêmes conditions expérimentales appliquées au (+)-modafinil conduisent à l'obtention d'un spectre de diffraction X identique.

### - Exemple 3 : Recristallisation dans le méthanol

a) 1 g de (-)-modafinil est ajouté dans 7 ml de méthanol chauffé au reflux jusqu'à dissolution complète. Le mélange réactionnel est précipité en ajoutant 6 ml d'eau à 1 °C. La suspension est maintenue sous agitation pendant 1 mn et ensuite filtrée sur un verre fritté (N° 3). Le solide isolé est séché pour conduire à la forme I du (-)-modafinil identifié par son spectre de Diffraction X. Rendement 55%.
b) Les mêmes conditions expérimentales appliquées au (+)-modafinil conduisent à l'obtention d'un spectre de diffraction X identique.

### - Exemple 4 : Recristallisation dans le méthanol (2^{e} exemple)

a) 2,5 g de (-)-modafinil sont ajoutés à 90 ml de méthanol chauffé au reflux jusqu'à dissolution complète du (-)-modafinil. La solution limpide est ajoutée à 200 ml d'eau à 1°C et laissée sans agitation pendant 10 mn. Le mélange réactionnel est filtré, et le solide récupéré est séché pour conduire à la forme I du (-)-modafinil identifié par son spectre de Diffraction X. Rendement 78%.
b) Les mêmes conditions expérimentales appliquées au (+)-modafinil conduisent à l'obtention d'un spectre de diffraction X identique.

### - Exemple 5 : Recristallisation dans le dioxanne 1-4

a) Dans un ballon de 50 mL, on introduit 20 mL de dioxanne 1-4 que l'on porte à reflux. 2 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de - 0,5°C/min jusqu'à 20°C. Les cristaux obtenus sont filtrés sur verre fritté et identifiés comme étant de la forme I par son spectre de Diffraction X. Rendement 51%.
b) Les mêmes conditions expérimentales appliquées au (+)-modafinil conduisent à l'obtention d'un spectre de diffraction X identique.

### - Exemple 6 : Recristallisation dans un mélange d'ortho, méta et para xylène

a) Dans un ballon de 250 mL, 180 mL d'un mélange d'ortho, méta et para xylène sont introduits et portés à reflux. 0,5 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de -0,5°C/min jusqu'à 15°C. Les cristaux obtenus sont filtrés sur verre fritté et identifiés comme étant de la forme I par son spectre de Diffraction X. Rendement 26%.
b) Les mêmes conditions expérimentales appliquées au (+)-modafinil conduisent à l'obtention d'un spectre de diffraction X identique.

### - Exemple 7 : Recristallisation dans l'acétate d'éthyle

a) Dans un ballon de 250 mL, on introduit 100 mL d'acétate d'éthyle que l'on porte à reflux; 2 g de (-)-modafinil sont ajoutés afin d'obtenir la saturation; l'agitation est assurée par un barreau magnétique (300 Tr/min). L'ensemble est refroidi après solubilisation totale du (-)-modafinil avec une rampe de refroidissement de -0,5°C/mn jusqu'à 20°C. Les cristaux obtenus sont filtrés sur verre fritté et identifiés comme étant de la forme I par son spectre de Diffraction X. Rendement 66%.
b) le (+)-modafinil (3 g) a été solubilisé à reflux dans l'acétate d'éthyl (100 ml). Après refroidissement par trempe dans un bain d'eau et de glace pendant 30 minutes, le milieu a été filtré puis séché à l'étuve à 50°C et sous vide. Le produit cristallisé a été identifié par son spectre de diffraction X sur poudre comme étant le polymorphe de forme I du (+)-modafinil.

### - Exemple 8 : à partir d'autres formes polymorphiques

a) Les CRL40982 forme IV (0,5 g) et CRL40982 forme II (0,5 g) donnent la forme I par chauffage à 100°C.
   De plus, la forme I pure du (-)-modafinil peut-être préparée par réempatage d'un mélange de (-)-modafinil forme I (0,5 g) et forme II (0,5 g) et forme III (0,5 g) dans l'acétone (20 ml) pendant un temps suffisant pour obtenir une transformation complète (3 jours).
   Dans les deux procédures, la forme I a été identifiée par son spectre de diffraction X obtenu sur poudre.
b) La mise en oeuvre du (+)-modafinil (CRL 40983) dans les mêmes conditions conduit aux mêmes résultats.

### - Exemple 9 : à partir du solvate d'acétonitrile

a) 1 g de solvate d'acétonitrile de (-)-modafinil chauffé à 100°C pendant 8 heures se transforme en un solide blanc identifié comme étant du (-)-modafinil forme I par son spectre de diffraction X sur poudre.
b) La mise en oeuvre du (+)-modafinil (CRL 40983) dans les mêmes conditions conduit aux mêmes résultats.

### - Exemple 10 : à partir du solvate de carbonate de monodiméthyle

a) 1 g de solvate carbonate de monodiméthyle de (-)-modafinil chauffé à 110°C pendant 16 heures se transforme en un solide blanc identifié comme étant du (-)-modafinil forme I par son spectre de diffraction X sur poudre.
b) La mise en oeuvre du (+)-modafinil (CRL 40983) dans les mêmes conditions conduit aux mêmes résultats.

### EXEMPLES 11 A 12 DE RÉFÉRENCE: PREPARATION DES SOLVATES DU (-)-MODAFINIL ET DU (+)-MODAFINIL

### - Exemple 11 de référence: Préparation du solvate de diméthylcarbonate du (-)-modafinil

a) 2 g de (-)-modafinil sont ajoutés à 20 ml de diméthylcarbonate et chauffés au reflux. Le mélange réactionnel est agité pendant 10 mn jusqu'à la dissolution complète du (-)-modafinil. La solution est refroidie lentement (-0,5°C/mn) jusqu'à 10°C sous agitation. Le mélange réactionnel est ensuite filtré sur un verre fritté (N° 3). L'analyse du solvate de modafinil diméthylcarbonate montre une masse d'environ 24 % en partant d'environ 50°C jusqu'à 110°C. La stoechiométrie du solvate de diméthylcarbonate est donc 1-1. Il s'agit donc d'un solvate vrai, identifié comme étant le solvate de diméthylcarbonate du (-)-modafinil par son spectre de diffraction X sur poudre. Rendement 88%.
b) Les mêmes conditions expérimentales appliquées au (+)-modafinil conduisent à l'obtention d'un spectre de diffraction X identique.

### - Exemple 12 de référence: Préparation du solvate d'acétonitrile du (-)-modafinil

a) Des cristaux de (-)-modafinil de forme polymorphique I sont suspendus dans de l'acétonitrile pendant 3 jours à 20°C. Le solide récupéré est identifié comme un solvate d'acétonitrile par diffraction aux rayons X. Le solvate correspond à un solvate vrai de stoechiométrie : 1-1. identifié comme étant le solvate d'acétonitrile du (-)-modafinil par son spectre de diffraction X sur poudre. Rendement 92%.
b) Les mêmes conditions expérimentales appliquées au (+)-modafinil conduisent à l'obtention d'un spectre de diffraction X identique.

### EXEMPLES 13 A 14 : STRUCTURES CRISTALLINES

### - Exemple 13 : Structure de l'acide modafinique

Des cristaux du modafinil ont été obtenus dans l'acétone. Cette phase a les caractéristiques suivantes :
- Hexagonal P3₁ ou P3₂ suivant l'énantiomère, le modafinil est donc un conglomérat ;
- a = 9,55, b = 9,55, c = 13,14 Å
- α = 90.000, β = 90.000, γ =120.000°

Les intensités diffractées ont été mesurées à l'aide d'un diffractomètre automatique SMART APEX (Brucker) à 20°C.

La structure a été résolue avec la suite de logiciels Saintplus, Sadabs, Shelxs.

Il est à souligner le caractère inusuel de ce groupe d'espace pour les molécules chirales organiques.

Dans la maille cristalline, le motif se répète trois fois, soit encore Z = 1. Ces molécules sont reliées entre elles par des liaisons hydrogène, via les fonctions acide et sulfoxide. On peut remarquer que les interactions les plus fortes (les liaisons hydrogène) s'enroulent autour de l'axe hélicoïdal ternaire suivant la direction cristallographique z.

### - Exemple 14 : Structure du (-) et (+)-modafinil forme I

La structure cristalline du (+) modafinil de forme I, identifiée comme identique à celle du (-) modafinil forme I, a été déterminée. Elle possède les caractéristiques suivantes :
- Système cristallin = monoclinique ;
- Groupe d'espace = P2₁
- a = 5.6938, b = 26.5024, c= 9.3346 Å
- β=105.970°

Les intensités diffractées ont été mesurées à l'aide d'un diffractomètre automatique SMART APEX (Brucker) à 20°C.

## Revendications

1. Forme polymorphique de l'énantiomère lévogyre ou dextrogyre du modafinil, désignée forme I, **caractérisée en ce que** (i) elle produit un spectre de diffraction X comprenant des raies d'intensité aux distances réticulaires : 8,54 ; 4,27 ; 4,02 ; 3,98 (À) ou **en ce que** (ii) elle produit un spectre de diffraction X comprenant des raies d'intensité aux valeurs d'angle 2 theta : 15,4 ; 31,1 ; 33,1 et 33,4 (degrés), mesurées en utilisant un diffractomètre Miniflex Rigaku (Elexience) en utilisant une radiation de chrome, l'erreur pour les valeurs 2 theta étant de ± 0,2 degrés 2 theta.

2. Forme polymorphique selon la revendication 1, **caractérisée en ce qu'**elle produit un spectre de diffraction X comprenant des raies d'intensité aux distances réticulaires : 8,54 ; 4,27 ; 4,02 ; 3,98 (À).

3. Forme polymorphique selon la revendication 2, **caractérisée en ce qu'**elle produit un spectre de diffraction X comprenant de plus des raies d'intensité aux distances réticulaires : 13,40 ; 6,34; 5,01 ; 4,68 ; 4,62 ; 4,44 ; 4,20 ; 4,15 ; 3,90 ; 3,80 ; 3,43 (À).

4. Forme polymorphique selon la revendication 1, **caractérisée en ce qu'**elle produit un spectre de diffraction X comprenant des raies d'intensité aux valeurs d'angle 2 theta : 15,4 ; 31,1 ; 33,1 et 33,4 (degrés), mesurées en utilisant un diffractomètre Miniflex Rigaku (Elexience) en utilisant une radiation de chrome, l'erreur pour les valeurs 2 theta étant de ± 0,2 degrés 2 theta.

5. Forme polymorphique selon la revendication 4, **caractérisée en ce qu'**elle produit un spectre de diffraction X comprenant de plus des raies d'intensité aux valeurs 2 thêta : 9,8 ; 20,8 ; 26,4 ; 28,3 ; 28,7 ; 29,9 ; 31,6 ; 32 ; 34,1 ; 35,1 et 39 (degrés), mesurées en utilisant un diffractomètre Miniflex Rigaku (Elexience) en utilisant une radiation de chrome, l'erreur pour les valeurs 2 theta étant de ± 0,2 degrés 2 theta.

6. Forme polymorphique selon l'une quelconque des revendications 1 à 5, qui est caractérisée comme suit :
| CRL 40982 FORME I | | |
|---|---|---|
| Theta (degrés) | d (A) | I/Io (%) |
| 9,8 ± 0,2 | 13,40 | 32 |
| 15,4 ± 0,2 | 8,54 | 87 |
| 20,8 ± 0,2 | 6,34 | 24 |
| 26,4 ± 0,2 | 5,01 | 14 |
| 28,3 ± 0,2 | 4,68 | 19 |
| 28,7 ± 0,2 | 4,62 | 16 |
| 29,9 ± 0,2 | 4,44 | 45 |
| 31,1 ± 0,2 | 4,27 | 100 |
| 31,6 ± 0,2 | 4,20 | 23 |
| 32 ± 0,2 | 4,15 | 14 |
| 33,1 ± 0,2 | 4,02 | 78 |
| 33,4 ± 0,2 | 3,98 | 84 |
| 34,1 ± 0,2 | 3,90 | 16 |
| 35,1 ± 0,2 | 3,80 | 15 |
| 39 ± 0,2 | 3,43 | 22 |
ces valeurs étant telles que mesurées en utilisant un diffractomètre Miniflex Rigaku (Elexience) en utilisant une radiation de chrome.

7. Composition pharmaceutique comprenant la forme polymorphique du (-) modafinil ou du (+)modafinil, désignée forme I, selon les revendications 1 à 6 et des excipients pharmaceutiquement acceptables.

8. Composition pharmaceutique consistant en la forme polymorphique dextrogyre ou lévogyre du modafinil désignée forme I, selon l'une quelconque des revendications 1 à 6, et en des excipients pharmaceutiquement acceptables.

9. Forme polymorphique du modafinil selon l'une quelconque des revendications 1 à 6, dans laquelle l'énantiomère est l'énantiomère (-).

10. Forme polymorphique du modafinil selon l'une quelconque des revendications 1 à 6, dans laquelle l'énantiomère est l'énantiomère (+).

11. Composition pharmaceutique comprenant une forme polymorphique du (-) modafinil ou une forme polymorphique du (+)modafinil selon la revendication 9 ou 10 et des excipients pharmaceutiquement acceptables.

12. Composition pharmaceutique consistant en une forme polymorphique du (-) modafinil ou une forme polymorphique du (+)modafinil selon l'une quelconque des revendications 9 et 10 et en des excipients pharmaceutiquement acceptables.

## Claims

1. Polymorphic form of the dextrorotatory or levorotatory enantiomer of modafinil, designated form I, **characterized in that** (i) it produces an X-ray diffraction spectrum comprising intensity peaks at the interplanar spacings: 8.54; 4.27; 4.02; 3.98 (Å); or (ii) it produces an X-ray diffraction spectrum comprising intensity peaks at 2 theta angle values: 15.4; 31.1; 33.1 and 33.4 (degrees), measured using a Miniflex Rigaku (Elexience) diffractometer using chromium radiation, the error in the 2 theta values being ± 0.2 degrees 2 theta.

2. Polymorphic form according to claim 1 **characterized in that** it produces an X-ray diffraction spectrum comprising intensity peaks at the interplanar spacings: 8.54; 4.27; 4.02; 3.98 (Å).

3. Polymorphic form according to claim 2 **characterized in that** it produces an X-ray diffraction spectrum further comprising intensity peaks at the interplanar spacings: 13.40; 6.34; 5.01; 4.68; 4.62 ; 4.44; 4.20; 4.15; 3.90; 3.80; 3.43 (Å).

4. Polymorphic form according to claim 1 **characterized in that** it produces an X-ray diffraction spectrum comprising intensity peaks at 2 theta angle values: 15.4; 31.1; 33.1 and 33.4 (degrees), measured using a Miniflex Rigaku (Elexience) diffractometer using chromium radiation, the error in the 2 theta values being ± 0.2 degrees 2 theta.

5. Polymorphic form according to claim 4 **characterized in that** it produces an X-ray diffraction spectrum further comprising intensity peaks at 2 theta values: 9.8; 20.8; 26.4; 28.3; 28.7; 29.9; 31.6; 32; 34.1; 35.1 and 39 (degrees), measured using a Miniflex Rigaku (Elexience) diffractometer using chromium radiation, the error in the 2 theta values being ± 0.2 degrees 2 theta.

6. Polymorphic form according to any one of claims 1 to 5, wherein the polymorphic form is characterised as follows:
| CRL 40982 FORM I | | |
|---|---|---|
| 2 Theta (degrees) | d (A) | I/Io(%) |
| 9.8 ± 0.2 | 13.40 | 32 |
| 15.4 ± 0.2 | 8.54 | 87 |
| 20.8 ± 0.2 | 6.34 | 24 |
| 26.4 ± 0.2 | 5.01 | 14 |
| 28.3 ± 0.2 | 4.68 | 19 |
| 28.7 ± 0.2 | 4.62 | 16 |
| 29.9 ± 0.2 | 4.44 | 45 |
| 31.1 ± 0.2 | 4.27 | 100 |
| 31.6 ± 0.2 | 4.20 | 23 |
| 32 ± 0.2 | 4.15 | 14 |
| 33.1 ± 0.2 | 4.02 | 78 |
| 33.4 ± 0.2 | 3.98 | 84 |
| 34.1 ± 0.2 | 3.90 | 16 |
| 35.1 ± 0.2 | 3.80 | 15 |
| 39 ± 0.2 | 3.43 | 22 |
these values being as measured using a Miniflex Rigaku (Elexience) diffractometer using chromium radiation.

7. Pharmaceutical composition comprising the polymorphic form of (-) modafinil or (+) modafinil, designated form I, according to claims 1 to 6 and pharmaceutically acceptable excipients.

8. Pharmaceutical composition consisting of the polymorphic form of dextrorotatory or levorotatory modafinil designated form I, according to any one of claims 1 to 6, and pharmaceutically acceptable excipients.

9. Polymorphic form of modafinil according to any one of claims 1 to 6, wherein the enantiomer is the (-) enantiomer.

10. Polymorphic form of modafinil according to any one of claims 1 to 6, wherein the enantiomer is the (+) enantiomer.

11. Pharmaceutical composition comprising a polymorphic form of (-) modafinil or a polymorphic form of (+) modafinil according to claim 9 or 10 and pharmaceutically acceptable excipients.

12. Pharmaceutical composition consisting of a polymorphic form of (-) modafinil or a polymorphic form of (+) modafinil according to any one of claims 9 and 10 and pharmaceutically acceptable excipients.

## Patentansprüche

1. Polymorphe Form des linksdrehenden oder rechtsdrehenden Enantiomers von Modafinil, die als Form I bezeichnet wird, **dadurch gekennzeichnet, dass** (i) sie ein Röntgenbeugungsspektrum mit Intensitätslinien bei den Netzebenenabständen 8,54, 4,27, 4,02 und 3,98 (Å) ergibt oder (ii) sie ein Röntgenbeugungsspektrum mit Intensitätslinien bei den 2-Theta-Winkel-Werten 15,4, 31,1, 33,1 und 33,4 (Grad) ergibt, gemessen unter Verwendung eines Diffraktometers der Bauart Miniflex Rigaku (Elexience) unter Verwendung von Chromstrahlung mit einem Fehler für die 2-Theta-Werte von ± 0,2 Grad 2 Theta.

2. Polymorphe Form nach Anspruch 1, **dadurch gekennzeichnet, dass** sie ein Röntgenbeugungsspektrum mit Intensitätslinien bei den Netzebenenabständen 8,54, 4,27, 4,02 und 3,98 (Å) ergibt.

3. Polymorphe Form nach Anspruch 2, **dadurch gekennzeichnet, dass** sie ein Röntgenbeugungsspektrum mit Intensitätslinien bei den Netzebenenabständen 13,40, 6,34, 5,01, 4,68, 4,62, 4,44, 4,20, 4,15, 3,90, 3,80 und 3,43 (Å) ergibt.

4. Polymorphe Form nach Anspruch 1, **dadurch gekennzeichnet, dass** sie ein Röntgenbeugungsspektrum mit Intensitätslinien bei den 2-Theta-Winkel-Werten 15,4, 31,1, 33,1 und 33,4 (Grad) ergibt, gemessen unter Verwendung eines Diffraktometers der Bauart Miniflex Rigaku (Elexience) unter Verwendung von Chromstrahlung mit einem Fehler für die 2-Theta-Werte von ± 0,2 Grad 2 Theta.

5. Polymorphe Form nach Anspruch 4, **dadurch gekennzeichnet, dass** sie ein Röntgenbeugungsspektrum mit Intensitätslinien bei den 2-Theta-Winkel-Werten 9,8, 20,8, 26,4, 28,3, 28,7, 29,9, 31,6, 32, 34,1, 35,1 und 39 (Grad) ergibt, gemessen unter Verwendung eines Diffraktometers der Bauart Miniflex Rigaku (Elexience) unter Verwendung von Chromstrahlung mit einem Fehler für die 2-Theta-Werte von ± 0,2 Grad 2 Theta.

6. Polymorphe Form nach einem der Ansprüche 1 bis 5, die folgendermaßen gekennzeichnet ist:
| CRL 40982 FORM I | | |
|---|---|---|
| 2 Theta (Grad) | d (Å) | I/Io (%) |
| 9,8 ± 0,2 | 13,40 | 32 |
| 15,4 ± 0,2 | 8,54 | 87 |
| 20,8 ± 0,2 | 6,34 | 24 |
| 26,4 ± 0,2 | 5,01 | 14 |
| 28,3 ± 0,2 | 4,68 | 19 |
| 28,7 ± 0,2 | 4,62 | 16 |
| 29,9 ± 0,2 | 4,44 | 45 |
| 31,1 ± 0,2 | 4,27 | 100 |
| 31,6 ± 0,2 | 4,20 | 23 |
| 32 ± 0,2 | 4,15 | 14 |
| 33,1 ± 0,2 | 4,02 | 78 |
| 33,4 ± 0,2 | 3,98 | 84 |
| 34,1 ± 0,2 | 3,90 | 16 |
| 35,1 ± 0,2 | 3,80 | 15 |
| 39 ± 0,2 | 3,43 | 22 |
wobei es sich bei diesen Werten um die unter Verwendung eines Diffraktometers der Bauart Miniflex Rigaku (Elexience) unter Verwendung von Chromstrahlung gemessenen Werte handelt.

7. Pharmazeutische Zusammensetzung, umfassend die polymorphe Form I von (-)-Modafinil oder (+)-Modafinil, die als Form I bezeichnet wird, nach den Ansprüchen 1 bis 6 und pharmazeutisch unbedenkliche Hilfsstoffe.

8. Pharmazeutische Zusammensetzung, bestehend aus der rechtsdrehenden oder linksdrehenden polymorphen Form I von Modafinil, die als Form I bezeichnet wird, nach den Ansprüchen 1 bis 6 und pharmazeutisch unbedenklichen Hilfsstoffen.

9. Polymorphe Form von Modafinil nach einem der Ansprüche 1 bis 6, wobei es sich bei dem Enantiomer um das (-)-Enantiomer handelt.

10. Polymorphe Form von Modafinil nach einem der Ansprüche 1 bis 6, wobei es sich bei dem Enantiomer um das (+)-Enantiomer handelt.

11. Pharmazeutische Zusammensetzung, umfassend eine polymorphe Form von (-)-Modafinil oder eine polymorphe Form von (+)- Modafinil nach Anspruch 9 oder 10 und pharmazeutisch unbedenkliche Hilfsstoffe.

12. Pharmazeutische Zusammensetzung, bestehend aus einer polymorphen Form von (-)-Modafinil oder einer polymorphen Form von (+)- Modafinil nach Anspruch 9 oder 10 und pharmazeutisch unbedenklichen Hilfsstoffen.
